# EUROPÄISCHE PATENTSCHRIFT

(11) **EP 2 288 311 B1**
(45) Veröffentlichungstag und Bekanntmachung des Hinweises auf die Patenterteilung: **07.05.2014**
(21) Anmeldenummer: 09737903.6
(22) Anmeldetag: 04.05.2009
(51) Int. Cl.: A61B 17/22, A61B 17/221, A61B 19/00

(54) **VORRICHTUNG ZUM ENTFERNEN VON KONKREMENTEN AUS KÖRPERGEFÄSSEN**
DEVICE FOR REMOVING CONCRETIONS FROM BODY VESSELS
DISPOSITIF POUR EXTRAIRE DES CONCRÉTIONS PRÉSENTES DANS DES VAISSEAUX DU CORPS

(30) Priorität: 02.05.2008 DE 102008021710; 28.11.2008 DE 102008059546
(43) Veröffentlichungstag der Anmeldung: 02.03.2011
(73) Patentinhaber: Acandis GmbH & Co. KG, 76327 Pfinztal (DE)
(72) Erfinder: CATTANEO, Giorgio, 76199 Karlsruhe (DE); BÜCHERT, Michael, 70193 Stuttgart (DE)
(74) Vertreter: Kilchert, Jochen
(86) Internationale Anmeldenummer: PCT/EP2009/003189
(87) Internationale Veröffentlichungsnummer: WO 2009/132859

(56) Entgegenhaltungen:
- EP-A- 1 632 188
- WO-A-99/39648
- WO-A-2005/087117
- DE-A1- 10 242 444
- US-A1- 2002 022 859
- US-A1- 2005 125 024
- US-A1- 2006 058 836

## Beschreibung

Die Erfindung betrifft eine Vorrichtung zum Entfernen von Konkrementen aus Körpergefäßen gemäß dem Oberbegriff des Anspruchs 1. Eine derartige Vorrichtung ist beispielsweise aus der WO 03/075793 A1 bekannt. Eine weitere Vorrichtung wird in der WO 99/39648 A beschrieben, aus der der Anspruch 1 abgeleitet wird.

Die Bildung von Konkrementen in Körpergefäßen kann dazu führen, dass das Körpergefäß durch das Konkrement vollständig oder zumindest teilweise verschlossen wird mit der Folge erheblicher gesundheitlicher Einschränkungen. Ein besonders hohes Risiko geht dabei von Thromben aus, d.h. von Blutgerinnseln, die sich intravasal bilden und das Blutgefäß partiell oder vollständig verschließen können. Wenn der Verschluss eine Arterie betrifft, werden die stromabwärts angeordneten Gewebeareale nicht mehr oder in unzureichendem Maß mit Sauerstoff und anderen Nährstoffen versorgt, was als Ischämie bezeichnet und ein Gewebesterben zur möglichen Folge hat.

Derzeit werden Thromben in menschlichen Arterien üblicherweise durch die medikamentöse Thrombolyse behandelt, wobei die Blutgerinnsel mit Hilfe von Medikamenten aufgelöst werden. Die Voraussetzung für diese Therapie ist, dass die Behandlung spätestens drei Stunden nach Auftreten der Symptomatik erfolgt. Zusätzlich zu diesem schmalen Zeitfenster hat die medikamentöse Thrombolyse den Nachteil, dass die Behandlung nicht nur lokal im Bereich des Thrombus, sondern auch in anderen Bereichen, beispielsweise in nahen Hirnbereichen wirkt. Durch die Hemmung der Blutgerinnung besteht die Gefahr von Blutungskomplikationen, insbesondere das Risiko, dass durch die Therapie eine Hirnblutung ausgelöst wird.

Eine Alternative zur medikamentösen Therapie ist die mechanische Entfernung von Thromben aus Blutgefäßen, bei der beispielsweise eine Vorrichtung gemäß der eingangs genannten WO 03/075793 A1 eingesetzt wird.

Dieser darin beschriebene Retriever umfasst einen aus Stegen mit einer Gitterstruktur gebildeten Käfig, in dem ein Thrombus gefangen und aus dem Körper abgeführt werden kann. Dazu ist der Käfig am proximalen Ende mit einem Betätigungsdraht verbunden, der den Käfig aus dem Katheter in das Blutgefäß schiebt bzw. diesen zusammen mit dem im Käfig gefangenen Thrombus in den Katheter zurückzieht. Der Käfig weist zwei Kammern mit unterschiedlichen Durchmessern auf, wobei die distale Kammer des Käfigs mit einem kleineren Durchmesser als die proximal angeordnete Kammer ausgebildet ist. Die engere distale Kammer ist schlauchförmig ausgebildet und weitet sich zu dem eigentlichen Aufnahmeabschnitt des Käfigs auf (proximale Kammer). Die Funktion der engeren distalen Kammer besteht darin, ein Gerinnsel im Blutgefäß greifen zu können, dessen Außendurchmesser kleiner ist, als der Innendurchmesser der größer dimensionierten Aufnahmekammer. Neben der Greiffunktion hat die enge schlauchförmige Kammer die Funktion, einen in der Aufnahmekammer gefangenen Thrombus zurückzuhalten.

Der Retriever gemäß WO 03/075793 A1 hat den Nachteil, dass die enge schlauchförmige Kammer den Transport des Thrombus in proximaler Längsrichtung im Retriever erschwert bzw. verhindert. Gerade ein größerer Thrombus gelangt nicht in die eigentlich zur Aufnahme des Thrombus vorgesehene größere Aufnahmekammer, sondern wird in der engen schlauchartigen Kammer gehalten und dort stark verformt, wodurch sich Partikel vom Thrombus lösen und in die Blutbahn gelangen können mit der möglichen Folge neuer Verschlüsse. Außerdem besteht die Gefahr, dass ein Thrombus mit einem größeren Durchmesser als die distale Kammer durch eine Vorschubbewegung des Retrievers in distaler Richtung vor sich her geschoben und nicht von der distalen Kammer aufgenommen wird. Der Thrombus kann dabei von der distalen Außenkante des Retrievers aufgespießt und verletzt werden, wodurch es wiederum zur Ablösung von Partikeln aus dem Thrombus kommen kann.

Der Erfindung liegt die Aufgabe zugrunde, eine Vorrichtung zum Entfernen von Konkrementen aus Körpergefäßen gemäß dem Oberbegriff des Anspruchs 1 so zu verbessern, dass eine einfache und sichere Handhabung der Vorrichtung beim Entfernen von Konkrementen aus Körpergefäßen ermöglicht und das gesundheitliche Risiko für den Patienten verringert wird.

Erfindungsgemäß wird diese Aufgabe durch den Gegenstand des Anspruchs 1 gelöst. Vorleilhafte Weiterbildungen der Erfindung sind Gegenstand der abhängigen Ansprüche.

Die Erfindung beruht auf dem Gedanken, eine Vorrichtung zum Entfernen von Konkrementen aus Körpergefäßen mit wenigstens einem komprimierbaren Aufnahmeelement zur Aufnahme von Konkrementen und einer Zufuhreinrichtung zur Führung des komprimierten Aufnahmeelements anzugeben. Dem Aufnahmeelement sind eine Betätigungseinrichtung und ein Verschlussmittel zugeordnet, wobei das Verschlussmittel in eine Offenstellung zum Einführen des Konkrements in das Aufnahmeelement und in eine Schließstellung zum Rückhalten des Konkrements im Aufnahmeelement überführbar ist. Die Betätigungseinrichtung ist erfindungsgemäß angepasst derart, dass diese im Gebrauch eine in Richtung des Aufnahmeelementes wirkende Kraft auf das zu entfernende Konkrement ausübt.

Die Erfindung hat den Vorteil, dass die Betätigungseinrichtung eine in Richtung des Aufnahmeelementes wirkende, insbesondere eine in proximaler Richtung wirkende Kraft auf das zu entfernende Konkrement ausübt und dadurch eine Verschiebung des Konkrements bzw. des Thrombus in Richtung des Aufnahmeelements bewirkt. Die Vorrichtung, insbesondere das Aufnahmeelement bleibt dabei während der Aufnahme des Thrombus ortsfest im Blutgefäß. Im Gegensatz dazu wird im Stand der Technik das Aufnahmeelement gegen bzw. über den ortsfest angeordneten Thrombus geschoben, so dass der Thrombus mit einer in distaler Richtung wirkender Kraft beaufschlagt wird, was dazu führen kann, dass der Thrombus in distaler Richtung verschoben wird.

Das Konkrement wird somit durch die in proximaler Richtung wirkende Betätigungseinrichtung sicher bei Offenstellung des Verschlussmittels in das Aufnahmeelement gezogen bzw. gedrückt und dort durch die Schließstellung des Verschlusselementes gehalten. Der Thrombus kann dann einfach aus dem Körpergefäß mittels der erfindungsgemäßen Vorrichtung entfernt werden.

Bei der Erfindung weist das Aufnahmeelement eine fluiddichte Abdeckung auf, die einen durch das zu entfernende Konkrement verschließbaren, insbesondere proximalen Hohlraum bildet, wobei die Betätigungseinrichtung im Gebrauch mit der Abdeckung und/oder dem Hohlraum in Wirkverbindung steht derart, dass im Hohlraum zum Bewegen, insbesondere zum Lösen des Konkrements ein anderer Druck einstellbar ist als außerhalb des Hohlraums.

Diese Ausführungsform hat den Vorteil, dass das Konkrement durch Einstellung eines Unterdrucks im Hohlraum, d.h. eines im Hohlraum niedrigeren Drucks als außerhalb des Hohlraums herrschenden physiologischen Drucks, in den Hohlraum bzw. das Aufnahmeelement eingesaugt bzw. durch den auf das Konkrement wirkenden höheren physiologischen Druck in den Hohlraum geschoben wird. Insofern wird durch die Betätigungseinrichtung eine in Richtung des Aufnahmeelementes wirkende Kraft (verursacht durch die Druckdifferenz auf beiden Seiten des Konkrements) auf das zu entfernende Konkrement erzeugt. Dabei ist es auch möglich, dass zumindest temporär das Druckgefälle umgekehrt wird derart, dass im Hohlraum ein Überdruck, d.h. ein höherer Druck als der hinter dem, d.h. distal zum Thrombus herrschende physiologische Druck erzeugt wird, so dass das Konkrement zum Lösen kurzzeitig mit einer in distaler Richtung wirkenden Kraft beaufschlagt wird. Zum Einsaugen des Konkrements in das Aufnahmeelement wird dann wieder ein Unterdruck im Hohlraum eingestellt.

Zur Bildung des Hohlraumes begrenzt das Aufnahmeelement einen in Axialrichtung, insbesondere in distaler Axialrichtung offenen Raum, der im Gebrauch durch das zu entfernende Konkrement verschließbar ist. Die Begrenzung des Raumes, bzw. Hohlraumes in der anderen Axialrichtung, insbesondere in proximaler Axialrichtung erfolgt durch die fluiddichte Abdeckung. Dabei ist die fluiddichte Abdeckung so angepasst, bzw. erstreckt sich soweit in distaler Richtung des Aufnahmeelements, dass die Abdeckung mit der Gefäßwand im Gebrauch fluidicht abschließt. Dadurch erfolgt eine in einer Richtung, insbesondere in proximaler Richtung wirkende Abdichtung des Gefäßes. Die Abdichtung in der anderen Richtung, insbesondere in distaler Richtung wird vom Konkrement übernommen, das an der Gefäßwand anliegt und das Gefäß verschließt.

Für den Verschluss des Hohlraums durch das Konkrement ist es nicht erforderlich, dass das Konkrement direkt am abgedichteten Aufnahmeelement anliegt. Es genügt, wenn der Querschnitt des dem Aufnahmeelement nachgeordneten Gefäßabschnittes und somit die Querschnittsfläche der distalen Öffnung des Aufnahmeelements vom Konkrement überdeckt wird, wobei das Konkrement geringfügig beabstandet vom Aufnahmeelement angeordnet sein kann. Ein hermetischer Gefäßverschluss durch das Konkrement ist nicht erforderlich, um das Druckgefälle zwischen der distalen und der proximalen Seite des Konkrements aufzubauen. Es genügt, wenn die durch das Konkrement eventuell nachströmende Blutmenge so gering ist, dass sich ein Druckausgleich mit Zeitverzögerung einstellt.

Somit bildet das Aufnahmeelement bzw. die fluiddichte Abdeckung im Zusammenwirken zumindest mit dem zu entfernenden Konkrement einen insbesondere proximalen Hohlraum. Durch die Betätigungseinrichtung, die dem Aufnahmeelement zugeordnet ist und im Gebrauch mit der Abdeckung und/oder dem Hohlraum in Wirkverbindung steht, kann im Hohlraum ein anderer Druck eingestellt werden, als außerhalb des proximalen Hohlraums, insbesondere auf der distalen Seite des zu entfernenden Konkrements. Dadurch wird das Konkrement mit einem hydraulischen Druck beaufschlagt, der zum Bewegen, insbesondere zum Lösen des Konkrements führt.

Unter dem Begriff Druck werden im Rahmen der vorliegenden Anmeldung nicht nur positive Drücke (Überdruck), sondern auch negative Drücke (Unterdruck) verstanden. Dabei ist ein Überdruck ein Druck, der höher ist als der im Gefäß herrschende Druck. Entsprechend ist ein Unterdruck ein Druck, der niedriger ist als der im Gefäß herrschende Druck. Bei der im Stand der Technik auftretenden möglichen Kollabierung des Gefäßes ist der Unterdruck so ausgeprägt, dass der transmurale Druck negativ wird.

Die Erfindung eignet sich insbesondere zum Entfernen von Gerinnseln bzw. Thromben aus Blutgefäßen. Obwohl nachfolgend die Erfindung am Beispiel der Entfernung von Thromben beschrieben wird, werden auch andere Einsatzmöglichkeiten der Vorrichtung, die das Entfernen von Konkrementen bzw. Objekten aus Körperhohlräumen betreffen, von der Erfindung umfasst.

Die Raumbezeichnungen bzw. Richtungsangaben, insbesondere die Bezeichnungen distal und proximal, beziehen sich auf die Position des Anwenders, insbesondere Arztes, der die Vorrichtung bedient. Distal angeordnete Objekte sind demnach bezüglich eines proximal angeordneten Objekts weiter vom Anwender bzw. Bediener der Vorrichtung entfernt angeordnet.

Das Betätigungselement kann eine Saug- und/oder Druckeinrichtung umfassen, die mit dem Hohlraum fluidverbunden oder fluidverbindbar ist. Dadurch wird auf einfache Weise die Druckänderung im Hohlraum bzw. im Aufnahmeelement erreicht, beispielsweise indem Fluid in den Hohlraum gepumpt bzw. Fluid aus dem Hohlraum abgesaugt wird.

Alternativ oder zusätzlich zu der hydraulischen Druckeinstellung mit der Saug- und/oder Druckeinrichtung kann das Betätigungselement einen distal zum Konkrement positionierbaren Schirm umfassen, der in Richtung des Aufnahmeelements bewegbar ist. Mit Hilfe des Schirms kann eine mechanische Kraft auf das Konkrement in Richtung des Aufnahmeelements, d.h. in proximaler Richtung aufgebracht werden. Es ist auch denkbar, den Schirm derart mit der Saug- und/oder Druckeinrichtung zu verbinden, dass auf der distalen Seite des Konkrements, d.h. zwischen Schirm und Konkrement ein Überdruck einstellbar ist, so dass auf das Konkrement eine hydraulische Kraft in proximaler Richtung wirkt.

Die Ausgestaltung der hydraulischen Druckbeaufschlagung auf das Konkrement ist in der weiteren Anmeldung mit dein Titel "Vorrichtung zum Entfernen von Konkrementen aus Körpergefäßen" offenbart, die am selben Tag wie die vorliegende Anmeldung eingereicht wurde und auf den selben Anmelder zurückgeht. Der Inhalt dieser Anmeldung wird durch Verweis vollumfänglich in die vorliegende Anmeldung aufgenommen.

Weiterhin weist das Verschlussmittel einen selbstschließenden Verschlussbereich auf, der zwischen einem distalen Einlassbereich und einem proximalen Auslassbereich des Verschlussmittels angeordnet ist, wobei der Einlassbereich zur Aufnahme des zu entfernenden Konkrements angepasst und der Auslassbereich zum Aufnahmeelement hin geöffnet ist. Das Verschlussmittel umfasst somit drei diskrete, in axialer Richtung nachgeordnete Bereiche mit unterschiedlichen Funktionen. Der distale Einlassbereich dient dazu, den proximalen Teil des Konkrements zu fangen und in die Nähe des Verschlussbereichs zu bringen. Dazu ist der Einlassbereich zur Aufnahme des zu entfernenden Konkrements angepasst und weist eine entsprechende Aufnahmekontur auf. Die Relativbewegung zwischen Konkrement und Einlassbereich wird, wie erläutert, durch eine Bewegung des Konkrements, verursacht durch die Betätigungseinrichtung, hervorgerufen.

Der dem distalen Einlassbereich axial nachgeordnete selbstschließende Verschlussbereich ist durch eine Bewegung des Konkrements betätigbar derart, dass das Verschlussmittel in eine Offenstellung zum Einführen des Konkrements in das Aufnahmeelement überführbar ist. Der selbstschließende Verschlussbereich weist dazu generell eine andere Form bzw. Dimensionierung, insbesondere im Hinblick auf den Durchmesser auf als der Einlassbereich.

Der dem selbstschließende Verschlussbereich axial nachgeordnete Auslassbereich des Verschlussmittels dient dazu, das Konkrement dem Aufnahmeelement zuzuführen. Dazu ist der Auslassbereich zum Aufnahmeelement hin geöffnet. Durch die in axialer Richtung nachgeordneten unterschiedlichen Bereiche des Verschlussmittels, insbesondere durch die Kopplung des Verschlussbereichs mit dem zur Aufnahme des Konkrements angepassten Einlassbereich wird im Gegensatz zum Stand der Technik die Einführung des Konkrements in das Aufnahmeelement vereinfacht.

Das Verschlussmittel, insbesondere der Verschlussbereich kann zumindest abschnittsweise eine Verjüngung mit einem veränderbaren Querschnittsdurchmesser umfassen, wobei die Verjüngung im Ruhezustand einen kleineren Querschnittsdurchmesser aufweist als der Einlassbereich des Verschlussmittels. Mit anderen Worten ist der Querschnittsdurchmesser des Einlassbereichs größer als der Querschnittsdurchmesser der Verjüngung im Ruhezustand. Im Gegensatz dazu ist der schlauchartige engere Bereich des Retrievers gemäß WO 03/075793 A1 zylinderförmig mit konstantem Querschnittsdurchmesser ausgebildet, der sich nur zum Aufnahmeelement hin erweitert. Da bei der erfindungsgemäßen Ausführungsform die Verjüngung im Ruhezustand einen kleineren Querschnittsdurchmesser aufweist als der Einlassbereich des Verschlussmittels, kann der Einlassbereich so dimensioniert sein, dass dieser besonders gut den proximalen Teil des Konkrements aufnimmt, ohne dass dabei die Verschlussfunktion der Verjüngung im Ruhezustand beeinträchtigt wird.

Bei einer besonders bevorzugten Ausführungsform ist das Verschlussmittel doppelttrichterförmig ausgebildet, wobei zwei trichterartige Abschnitte miteinander verbunden sind und im Halsbereich zwischen den Abschnitten die Verjüngung bilden. Dadurch wird sowohl der Eintritt des Konkrements in die Vorrichtung im Einlassbereich als auch die Überführung des Konkrements vom Verschlussmittel in das Aufnahmeelement erleichtert. Außerdem ist die Doppeltrichterform des Verschlussmittels herstellungstechnisch günstig. Ein weiterer Vorteil der Doppeltrichterform besteht darin, dass die durch die Betätigungseinrichtung aufgebrachte Kraft zum Bewegen des Konkrements in Richtung des Aufnahmeelementes durch die Schließbewegung der Verjüngung bzw. des Halsbereichs zwischen den beiden trichterartigen Abschnitten mechanisch unterstützt wird, wenn das Konkrement fast vollständig durch den Halsbereich hindurchgetreten ist. Das bedeutet, dass das Konkrement durch die Schließbewegung der Verjüngung in den Aufnahmeabschnitt eingeschoben wird. Außerdem legt sich das doppeltrichterförmige Verschlusselement eng, insbesondere abdichtend um das Konkrement, wodurch die Saugwirkung des Betätigungselements bzw. der Saug- und/oder Druckeinrichtung verbessert wird.

Das Verschlussmittel kann im Bereich der Verjüngung eine Verschlussöffnung bilden, die mit dem Aufnahmeelement fluchtet, insbesondere zum Aufnahmeelement koaxial angeordnet ist, so dass das Konkrement ungehindert in das Aufnahmeelement durch die Verschlussöffnung gleiten kann.

Es hat sich als zweckmäßig erwiesen, wenn das Verhältnis des Innendurchmessers der Verjüngung zum Innendurchmesser des Einlassbereichs im Ruhezustand höchstens 0,8, insbesondere höchstens 0,5, insbesondere höchstens 0,1, insbesondere höchstens 0,5, insbesondere höchstens 0,02 beträgt. Die Verjüngung kann im Ruhezustand eine Querschnittsfläche von höchstens 10 mm², insbesondere höchstens 5 mm², insbesondere höchstens 1 mm², insbesondere höchstens 0,5 mm², insbesondere höchstens 0,2 mm², insbesondere höchstens 0,1 mm² umfassen.

Die Aufweitung des Verschlussmittels im Bereich der Verjüngung beim Eintritt des Konkrements in die Vorrichtung wird dadurch erleichtert, dass das Verschlussmittel zumindest im Bereich der Verjüngung wenigstens eine Druckausgleichsöffnung aufweist. Dadurch wird erreicht, dass im Bereich zwischen der Verjüngung und dem Gefäß der selbe Druck herrscht, wie im Aufnahmeelement.

Das Verschlussmittel kann eine Axiallänge von höchstens 10 mm, insbesondere höchstens 8 mm, insbesondere höchstens 5 mm, insbesondere höchstens 2 mm aufweisen. Die fluiddichte Abdeckung des Aufnahmeelementes kann sich mindestens bis vor den Auslassbereich erstrecken. Dadurch wird erreicht, dass die fluiddichte Abdichtung des Aufnahmeelements gegen die Gefäßwand bis zum Verschlussmittel gewährleistet ist.

Das Verschlussmittel kann im Wesentlichen eine geflochtene, insbesondere stentähnliche Gitterstruktur umfassen, die im Hinblick auf die Herstellung des Profils bzw. der Form des Verschlussmittels günstig ist. Vorzugsweise weist die Gitterstruktur, wenn geflochten, im Bereich der Verjüngung im Ruhezustand einen kleineren Flechtwinkel auf als im Bereich des Aufnahmeabschnitts. Dadurch wird eine gute Ausweitung der Verjüngung beim Durchtritt des Konkrements erreicht, ohne dass es dabei zu einer übermäßigen Längskomprimierung des Verschlussmittels kommt. Die radiale Ausdehnung des Verschlussmittels, insbesondere der Verjüngung überwiegt somit. Bei der geflochtenen Gitterstruktur kann vorzugsweise eine schraubenförmige Geometrie der Drähte der geflochtenen Gitterstruktur vorgesehen sein. Eine derartige geflochtene Struktur hat den Vorteil, dass eine geringe axiale Komprimierung, durch die schraubenförmige Geometrie der Drähte eine radiale Ausweitung hervorruft. Das bedeutet, dass eine axiale Komprimierung durch die vom Konkrement ausgeübte Kraft zu einer Ausweitung bzw. Aufweitung der Verjüngung führt, ohne dass Radialkräfte ausgeübt werden. Aufgrund des kleinen Flechtwinkels ist die Ausweitung besonders in der Anfangsphase der Aufweitung bzw. Aufschließung der Verjüngung, in der der Winkel am kleinsten ist, selbst bei kleinen Komprimierungen sehr ausgeprägt.

Das Verschlussmittel weist vorzugsweise im Einlassbereich und/oder im Auslassbereich Querschnittsänderung, insbesondere eine kontinuierliche Querschnittsänderung auf, wodurch eine gleitende ungehinderte Bewegung des Konkrements durch das Verschlussmittel hindurch verbessert wird.

Dem Verschlussmittel kann ein Stützelement zugeordnet sein, das radial außen bezogen auf das Verschlussmittel angeordnet ist und im Gebrauch eine Gefäßwand des Körpergefäßes zumindest im Bereich der Verjüngung stützt. Dadurch wird eine Verformung der Gefäßwand im Bereich der Verjüngung verhindert, die möglicherweise durch die Druckbeaufschlagung des Aufnahmeelements auftreten kann insbesondere, bevor das Konkrement in das Verschlussmittel eintritt. Dies ist insbesondere dann relevant, wenn das Verschlussmittel mit Druckausgleichöffnungen versehen ist, da dann der Unterdruck, d.h. ein Druck, der kleiner als der physiologische Druck ist, auch im Außenbereich des Verschlussmittels radial um die Verjüngung herum wirkt.

Bei einer weiteren bevorzugten Ausführungsform umfasst das Verschlussmittel eine Vielzahl von Drähten mit freien Enden, die am distalen Ende des Aufnahmeelements angeordnet sind und in der Ruheposition radial nach innen vorstehen. Dadurch wird alternativ zu dem bekannten Verschlussmittel gemäß WO 03/075793 A1 eine andere Rückhaltemöglichkeit geschaffen, um ein im Aufnahmeelement angeordnetes Konkrement dort zu halten. Diese Ausbildung des Verschlussmittels ist besonders einfach zu realisieren, insbesondere bei geflochtenen Strukturen. Dabei können die freien Enden in proximaler Richtung vorstehen und/oder in Umfangsrichtung des Aufnahmeelements gekrümmt sein. Die Drähte können dabei als Ventil oder Klappe wirken, die einen Eintritt des Konkrements in den Aufnahmebereich der Vorrichtung ermöglichen und den Austritt des Konkrements aus dem Aufnahmebereich verhindern.

Bei einer weiteren bevorzugten Ausführungsform sind die freien Enden mit einer weiteren fluiddichten Abdeckung, insbesondere einer flächigen Beschichtung verbunden, die eine Durchtrittsöffnung für das zu entfernende Konkrement, insbesondere eine schließbare Durchtrittsöffnung, bildet und eine Bewegung der freien Enden radial nach außen erlaubt. Dadurch wird die Rückhaltefunktion der Drähte bzw. des Verschlussmittels verbessert und darüber hinaus eine sichere Barriere geschaffen, die einen Austritt von sich eventuell vom gefangenen Thrombus lösenden Partikeln in distaler Richtung verhindert.

Das Verschlussmittel umfasst bei einer weiteren nicht erfindungsgemäßen Ausführungsform zwei trichterartige Abschnitte, die doppelkegelförmig angeordnet sind und die Verjüngung bilden, wobei die trichterartigen Abschnitte unterschiedliche Trichterwinkel aufweisen. Bezüglich einer im Bereich der Verjüngung angeordneten Querschnittsebene des Verschlussmittels sind die beiden trichterartigen Abschnitte somit asymmetrisch ausgebildet. Als Trichterwinkel wird im Rahmen der Erfindung der Winkel bezeichnet, der zwischen der Außenfläche des trichterartigen Abschnitts und der Rotationsachse des Verschlussmittels gebildet ist.

Die trichterartigen Abschnitte können einen distalen Einlassbereich und einen proximalen Auslassbereich bilden, wobei der distale Einlassbereich einen kleineren Trichterwinkel aufweist als der proximale Auslassbereich. Bei gleicher Grundfläche der beiden trichterartigen Abschnitte weist der distale Einlassbereich somit eine größere Längserstreckung auf als der proximale Auslassbereich. Durch den sich unter einem flacheren Winkel verjüngenden distalen Einlassbereich wird erreicht, dass beim Einsaugen bzw. Aspirieren eines Thrombus die Verjüngung des Verschlussmittels einfacher aufgeweitet wird. Insbesondere ist zur Aufweitung der Verjüngung eine relativ kleine Kraft erforderlich, die vom Thrombus beim Einsaugen auf die Verjüngung ausgeübt wird. Durch den relativ steilen Trichterwinkel des proximalen Auslassbereichs wird hingegen erreicht, dass der Thrombus im Aufnahmeelement sicher gehalten wird, da zum Aufweiten der Verjüngung durch Bewegung des Thrombus in distale Richtung eine größere Kraft erforderlich ist.

Das Verschlussmittel kann einen distalen Endabschnitt aufweisen, der radial nach außen geformt ist derart, dass der Einlassbereich im Wesentlichen trompetenartig ausgebildet ist. Das Verschlussmittel bzw. der distale Endabschnitt kann freie Drahtenden und/oder Drahtschlaufen oder andere Drahtabschlüsse aufweisen. Die Drahtenden können beschichtet sein.Im implantierten Zustand bzw. im Gebrauch der Vorrichtung wird mit dem trompetenartigen Einlassbereich ein fließender bzw. kontinuierlicher Übergang zwischen der Gefäßwand und dem Verschlussmittel erreicht, so dass sich der zu entfernende Thrombus beim Ansaugen nicht an den freien Drahtenden des Verschlussmittels bzw. des distalen Endabschnitts verhaken kann. Ferner bildet der radial nach außen geformte, distale Endabschnitt einen Schneidebereich, womit ein stark an der Gefäßwand anhaftender Thrombus gelöst werden kann. Dazu wird die Vorrichtung behutsam um ihre Rotationsachse gedreht und gleichzeitig unter kontinuierlicher Aspiration in distale Richtung auf den Thrombus zugeschoben, so dass der Schneidebereich den Thrombus von der Gefäßwand trennt.

Bei einer weiteren nicht erfindungegemäßen Ausführungsform umfasst das Aufnahmeelement einen proximalen Dichtungsabschnitt, der einen größeren Querschnittsdurchmesser aufweist als ein zylindrischer Abschnitt des Aufnahmeelements. Der Übergang zwischen dem proximalen Dichtungsabschnitt und dem zylindrischen Abschnitt des Aufnahmeelements ist vorzugsweise kontinuierlich bzw. fließend. Der proximale Dichtungsabschnitt gewährleistet im Gebrauch eine verbesserte Abdichtung des Aufnahmeelements gegenüber der Gefäßwand. Dabei erfolgt die Abdichtung auf relativ kleiner Fläche, wodurch der Flächendruck auf das Gefäß bzw. die Gefäßwand auf einen kleinen Gefäßabschnitt beschränkt und somit insgesamt reduziert wird. Bei Bewegung des Aufnahmeelements, beispielsweise beim Zurückziehen des Aufnahmeelements nach erfolgreicher Entfernung eines Thrombus, wird durch den Dichtungsabschnitt das Gefäß in einem relativ kleinen Bereich mit Scherkräften beaufschlagt, so dass die Belastung des Gefäßes bzw. der Gefäßwand reduziert wird. Bei Bewegung des Aufnahmeelements in distaler Richtung, d.h. beim Schieben des Aufnahmeelements, bewirkt der proximale Dichtungsabschnitt eine verbesserte Abdichtung gegenüber dem Gefäß, da der proximale Dichtungsabschnitt gestaucht wird, wodurch sich der Querschnittsdurchmesser des proximalen Dichtungsabschnitts erhöht.

Des Weiteren kann das Verschlussmittel wenigstens zwei Verjüngungen umfassen, die koaxial zueinander angeordnet sind, wobei eine proximal angeordnete Verjüngung eine kleinere Querschnittsfläche aufweist als eine distal angeordnete Verjüngung, die eine kleinere Querschnittsfläche aufweist als ein an einem distalen Ende des Verschlussmittels angeordneten Einlassbereich. Im Bereich der beiden Verjüngungen ist jeweils eine Verschlussöffnung gebildet, wobei der Querschnittsdurchmesser der Verschlussöffnung von distal nach proximal abnimmt, d.h. die Verengungen werden von distal beginnend nach proximal zunehmend enger. Unabhängig von der Größe des zu entfernenden Thrombus wird das Einsaugen des Thrombus dadurch vereinfacht. Beim Ansaugen verformt sich der Thrombus, der aus einem weichen, geleeartigen Material besteht, da der Unterdruck im Hohlraum zwischen Aufnahmeelement und Thrombus überwiegend im proximalen Bereich des Thrombus wirkt. Durch die mehreren Verjüngungen, deren Verschlussöffnungen in proximaler Richtung einen zunehmend kleineren Querschnittsdurchmesser aufweisen, wird der Thrombus mit relativ geringem Kraftaufwand abschnittsweise in das Aufnahmeelement hineingesogen. Ein Zerreißen des Thrombus wird somit vermieden. Ein weiterer Vorteil dieser Ausführungsform besteht darin, dass durch die mehreren Verjüngungen die Umfangsfläche des Aufnahmeelements, die die Gefäßwand berührt, verringert ist. Durch die kleinere Kontaktfläche zwischen Aufnahmeelement und Gefäßwand wird die Belastung der Gefäßwand reduziert.

Alle vorstehend genannten Merkmale betreffend die Ausgestaltung des Verschlussmittels werden auch unabhängig von der Betätigungseinrichtung, also im Zusammenhang mit der Vorrichtung ohne Betätigungseinrichtung offenbart.

Die Erfindung wird nachfolgend anhand von Ausführungsbeispielen mit weiteren Einzelheiten und unter Bezugnahme auf die beigefügten schematischen Zeichnungen näher erläutert. In diesen zeigen:
- Fig. 1: einen Längsschnitt durch eine Vorrichtung nach einem erfindungsgemäßen Ausführungsbeispiel, bei der das zu entfernende Konkrement distal von der Vorrichtung angeordnet ist;
- Fig. 2: die Vorrichtung gemäß Fig. 1, wobei das Konkrement teilweise vom Verschlussmittel aufgenommen ist;
- Fig. 3: die Vorrichtung gemäß Fig. 1, wobei das Konkrement sich vollständig im Bereich des Verschlussmittels befindet;
- Fig. 4: die Vorrichtung gemäß Fig. 1, wobei das Konkrement im Aufnahmeelement der Vorrichtung angeordnet ist;
- Fig. 5: die Vorrichtung gemäß Fig. 1 mit verformter Gefäßwand im Bereich des Verschlussmittels;
- Fig. 6: die Vorrichtung gemäß Fig. 1 mit Stützstruktur;
- Fig. 7: eine Vorrichtung nach einem weiteren erfindungsgemäßen Ausführungsbeispiel mit einem alternativen Verschlussmittel;
- Fig. 8: eine Vorrichtung nach einem weiteren erfindungsgemäßen Ausführungsbeispiel mit einer anderen Variante des Verschlussmittels; und
- Fig. 9-11: jeweils eine Vorrichtung nach einem weiteren erfindungsgemäßen Ausführungsbeispiel.

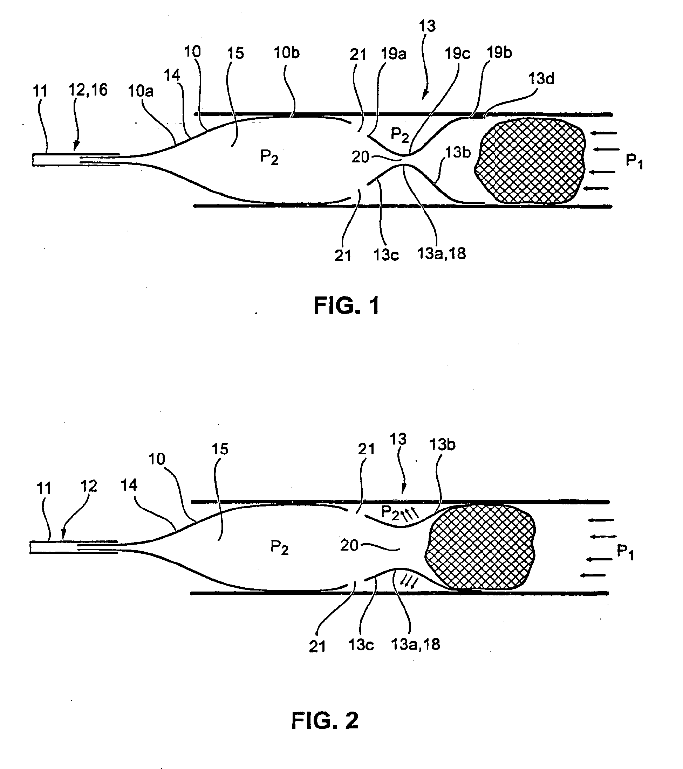

In Fig. 1 ist ein Beispiel für eine erfindungsgemäße Vorrichtung zum Entfernen von Konkrementen aus Körpergefäßen dargestellt, die besonders bevorzugt zur Entfernung von Thromben eingesetzt wird. Besonders geeignet ist die Vorrichtung zur cerebralen Behandlung von Thromben, da die Vorrichtung gute Crimpeigenschaften aufweist und entsprechend in Mikrokathetersysteme eingebracht werden kann. Die Vorrichtung ist auch zur Entfernung anderer Konkremente in Körpergefäßen oder anderen Körperhohlräumen geeignet.

Nachfolgend wird der Aufbau der Vorrichtung beschrieben, die ein komrimierbares, insbesondere crimpbares Aufnahmeelement 10 zur Aufnahme von Konkrementen sowie eine Zufuhreinrichtung 11, insbesondere einen Mikrokatheter, umfasst. Die Zufuhreinrichtung 11 ist zur Führung des komprimierten Aufnahmeelements 10 vorgesehen und bringt das komprimierte Aufnahmeelement in das Körpergefäß in die Nähe des zu entfernenden Konkrements ein.

Das Aufnahmeelement ist in den Figuren 1 bis 4 in expandiertem Zustand dargestellt und weist eine korbartige bzw. käfigartige Struktur auf. Aufbau und Form des Aufnahmeelements entsprechen der in der eingangs erwähnten Anmeldung "Vorrichtung zum Entfernen von Konkrementen aus Körpergefäßen" offenbarten Vorrichtung, auf die auch im Zusammenhang mit dem Aufnahmeelement bzw. den übrigen identischen Bauteilen Bezug genommen wird. Das Aufnahmeelement 10 ist trichterförmig ausgebildet und weist einen sich in proximaler Richtung verjüngenden trichterförmigen Abschnitt 10a auf. Der trichterförmige Abschnitt 10a ist in distaler Richtung mit einem zylinderförmigen Abschnitt 10b verbunden, insbesondere koaxial verbunden. Der Querschnittsdurchmesser des zylinderförmigen Abschnitts 10b entspricht im Wesentlichen dem Durchmesser des Blutgefäßes, so dass der zylinderförmige Abschnitt 10b im expandierten Zustand an der Gefäßwand anliegt und diese abstützt. Andere Formen des Aufnahmeelements sind möglich, die die Abstützfunktion erfüllen.

Das Aufnahmeelement 10 weist ferner eine fluiddichte Abdeckung 14 auf, die das Aufnahmeelement 10 zumindest soweit bedeckt, dass der zylindrische Abschnitt 10b des Aufnahmeelements fluiddicht an der Gefäßwand anliegt. Die Abdeckung 14 erstreckt sich somit mindestens bis zum zylindrischen Abschnitt 10b, insbesondere teilweise in den zylindrischen Abschnitt 10b hinein, insbesondere bis zum distalen Ende des zylindrischen Abschnitts 10b. Die Abdeckung 14 kann als freitragende Stützstruktur durch Sputtern oder ein anderes Beschichtungsverfahren hergestellt sein oder auf eine Gitterstruktur, insbesondere eine geflochtene oder geschnittene Gitterstruktur des Aufnahmeelements 10 aufgebracht sein, ebenfalls durch ein an sich bekanntes Beschichtungsverfahren.

Wie in Fig. 1 weiter dargestellt, bildet das Aufnahmeelement 10 im Inneren einen Hohlraum 15, der in Fluidverbindung mit dem Katheter bzw. der Zufuhreinrichtung 11 und einer zugehörigen Saug- und/oder Druckeinrichtung 16 steht. Die Saug- und/oder Druckeinrichtung 16 kann mittels einer (nicht dargestellten) Druckleitung mit dem Hohlraum 15 bzw. der Abdeckung 14 verbunden sein, wobei die Druckleitung mit dem Auf-nahmeelement 10 bzw. der fluiddichten Abdeckung 14 fluiddicht gekoppelt ist. Mittels der Saug- und/oder Druckeinrichtung 16 bzw. allgemein mittels der Betätigungseinrichtung 12, die dem Aufnahmeelement 10 zugeordnet ist, lässt sich eine in Richtung des Aufnahmeelementes 10 wirkende Kraft auf das zu entfernende Konkrement erzeugen, insbesondere dadurch, dass im Hohlraum 15 durch die Betätigungseinrichtung 12 ein anderer Druck einstellbar ist, als außerhalb des Hohlraums 15.

Dazu wird das im Hohlraum 15 befindliche Flüssigkeitsvolumen zumindest teilweise abgepumpt bzw. abgesaugt, so dass im abgedichteten Hohlraum 15 ein niedrigerer Druck als der physiologische Druck eingestellt wird, der auf der distalen Seite des Konkrements wirkt und dieses mit einer in Richtung des Aufnahmeelements 10 wirkenden Kraft beaufschlagt. Dadurch wird das Konkrement in Richtung des ortsfest angeordneten Aufnahmeelements 10 verschoben. Es ist möglich, zum besseren Lösen des Konkrements das Druckgefälle umzukehren, indem im Hohlraum 15 ein Überdruck, also ein höherer Druck als der physiologische Druck eingestellt wird, wodurch eine in distaler Richtung wirkende Kraft auf das Konkrement ausgeübt wird. Durch ein entsprechend zeitlich alternierendes Druckgefälle wird der Thrombus sicher und schonend von der Gefäßwand gelöst und als Ganzes in das Aufnahmeelement verschoben.

Dem Aufnahmeelement 10 ist ein Verschlussmittel 13 zugeordnet, das in eine Offenstellung zum Einführen des Konkrements in das Aufnahmeelement 10 und in eine Schließstellung zum Rückhalten des Konkrements im Aufnahmeelement 10 überführbar ist. Das Verschlussmittel 13 umfasst drei verschiedene Bereiche, die sich durch unterschiedliche Durchmesser bzw. durch eine unterschiedliche Ausrichtung unterscheiden und verschiedene Funktionen wahrnehmen. Das Verschlussmittel 13 weist insbesondere einen selbstschließenden Verschlussbereich 13a auf, sowie einen distalen Einlassbereich 13b und einen proximalen Auslassbereich 13c, die jeweils dem Verschlussbereich 13a zugeordnet sind. Der Einlassbereich 13b ist zur Aufnahme des zu entfernenden Konkrements angepasst und weist zumindest in der Ruheposition einen größeren Innendurchmesser auf, als der Verschlussbereich 13a in der Ruheposition. Der Auslassbereich 13c ist zum Aufnahmeelement 10 hin geöffnet und weist ebenfalls einen größeren Durchmesser auf als der Verschlussbereich 13a. Der Verschlussbereich 13a bildet eine Verjüngung 18 zwischen dem Einlassbereich 13b und dem Auslassbereich 13c, die einen veränderbaren Querschnittsdurchmesser umfasst. Die Verjüngung 18 ist hierzu flexibel ausgebildet und ermöglicht dadurch die Offen- bzw. Schließstellung des Verschlussmittels 13.

Konkret ist das Verschlussmittel 13 doppeltrichterförmig ausgebildet und weist zwei trichterartige Abschnitte 19a, 19b auf, die im Bereich des engsten Querschnitts miteinander verbunden sind. Der sich dabei bildende Halsbereich 19c zwischen den Abschnitten 19a, 19b entspricht der Verjüngung 18. Der Halsbereich 19c, bzw. die Verjüngung 18 hat eine axiale Erstreckung und ist ringförmig bzw. zylinderförmig ausgebildet. Die beiden trichterartigen Abschnitte 19a, 19b sind jeweils im Einlass- bzw. Auslassbereich 13b, 13c des Verschlussmittels angeordnet bzw. entsprechend diesen Bereichen.

Die Erfindung ist nicht auf die Doppeltrichterform des Verschlussmittels eingeschränkt, sondern umfasst auch andere Querschnittsformen des Einlass- und Auslassbereichs 13a, 13b, 13c des Verschlussmittels 13, bzw. in Form eines Konus bzw. Doppelkonus. Trichter- und Konusformen können kombiniert werden. Ferner ist es möglich, die beiden trichterartigen Abschnitte 19a, 19b bzw. den Einlass- und Auslassbereich 13b, 13c mit jeweils unterschiedlichen Innendurchmessern auszubilden, wobei der Innendurchmesser des Auslassbereichs 13c, beispielsweise größer als der Innendurchmesser des Einlassbereichs 13b sein kann, da dann die Dichtfunktion des Aufnahmeelements 10 gewährleistet ist. In Fig. 1 ist ferner zu erkennen, dass das Verschlussmittel 13 im Bereich der Verjüngung 18 eine Verschlussöffnung 20 bildet, die mit dem Aufnahmeelement 10 fluchtet, insbesondere zum Aufnahmeelement 10 koaxial angeordnet ist.

Wie in Fig. 1 weiter zu erkennen, geht der trichterförmige Auslassbereich 13c in einer kontinuierlichen Querschnittserweiterung bzw. allgemein Querschnittsänderung in das Aufnahmeelement 10 über bzw. ist mit diesem verbunden. Konkret ist der Auslassbereich 13c des Verschlussmittels 13 mit dem zylindrischen Abschnitt 10b des Aufnahmeelements 10 verbunden. Der ebenfalls trichterförmige Einlassbereich 13b weist einen zylindrischen Endabschnitt 13d auf, dessen Außendurchmesser so dimensioniert ist, dass dieser an der Gefäßwand anliegt. Der Übergang zwischen dem Einlassbereich 13b und dem Endabschnitt 13c erfolgt durch eine kontinuierliche Querschnittsänderung bzw. Querschnittserweiterung. In diesem Ausführungsbeispiel sind somit die Außendurchmesser des zylindrischen Endabschnitts 13c und des zylindrischen Abschnitts 10b des Aufnahmeelements 10 mit denselben Außendurchmessern dimensioniert. Unterschiedliche Durchmesser sind möglich.

Die kontinuierliche Querschnittsänderung kann zumindest abschnittsweise linear und/oder mit einer Wölbung erfolgen. Vorzugsweise an den axialen Übergängen des Einlassbereichs 13b bzw. des Auslassbereichs 13c zu den jeweils angrenzenden Bereichen ist die Wandung gekrümmt, um Kanten zu vermeiden, die das Gleiten des Konkrements beeinträchtigen könnten.

Die fluiddichte Abdeckung 14 erstreckt sich bis kurz vor den Auslassbereich 13c, d.h. bis zum proximalen Ende des Auslassbereichs 13c. Die fluiddichte Abdeckung 14 kann sich teilweise bis in den Auslassbereich 13c, insbesondere bis zur Verjüngung 18 erstrecken. Es ist auch möglich, dass die Abdeckung die komplette Vorrichtung bedeckt, d.h. dass sich die Abdeckung 14 bis zum distalen Ende des Verschlussmittels 13 erstreckt.

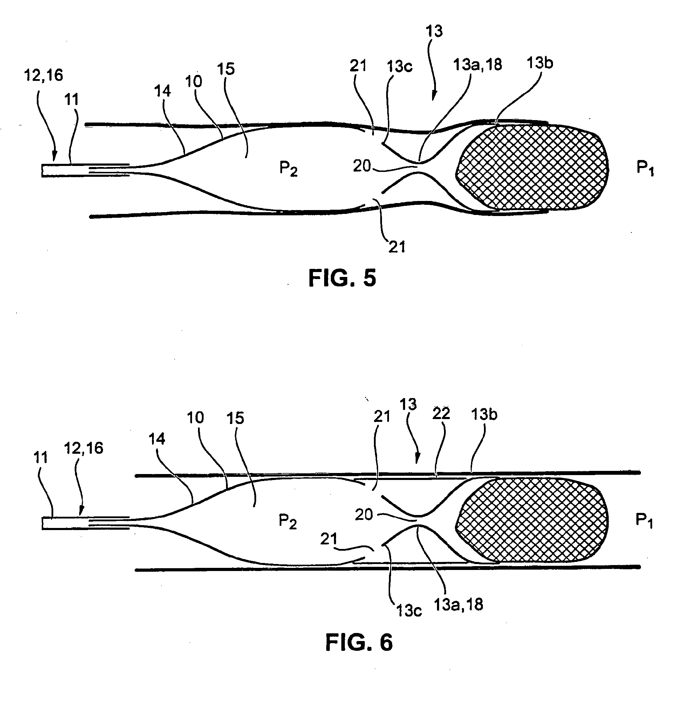

Wie in Fig. 1 ferner dargestellt, weist das Verschlussmittel 13 zumindest im Bereich der Verjüngung 18 wenigstens eine, insbesondere mehrere Druckausgleichsöffnungen 21 auf. Konkret ist die Druckausgleichsöffnung 21 im Bereich des Auslassbereichs 13c, insbesondere im proximalen Trichter angeordnet, d.h. proximal von der Verjüngung 18. Die Druckausgleichsöffnung 21 bzw. die Vielzahl der Druckausgleichsöffnungen 21 dient dazu, das Aufweiten der Verjüngung 18 zu unterstützen, wenn im Hohlraum 15 ein Unterdruck angelegt wird. Die Druckausgleichsöffnungen 21 sind distal etwas entfernt vom zylindrischen Abschnitt 10b angeordnet, damit diese nicht an der Gefäßwand anliegen, sondern frei durchströmbar sind. Bei der Beaufschlagung des Hohlraums 15 mit dem Unterdruck kann es zu einer Gefäßverformung im Bereich der Verjüngung kommen, da der Unterdruck aufgrund der Druckausgleichsöffnungen 21 auch in dem Umfangsbereich außerhalb der Verjüngung 18 wirkt (Fig. 5). Um diese Gefäßverformung zu vermeiden, ist beim Ausführungsbeispiel gemäß Fig. 6 eine axial sich erstreckende Stützstruktur bzw. ein Stützelement 22 dem Verschlussmittel 13 zugeordnet, das radial außen bezogen auf das Verschlussmittel 13 angeordnet ist. Im Gebrauch stützt das Stützelement 22 die Gefäßwand des Körpergefäßes im Bereich der Verjüngung 18. Das Stützelement 22 kann als stentartige Hülse ausgebildet sein, die den Außenumfang des Einlassbereiches 13b mit dem maximalen Außenumfang des Auslassbereiches 13c bzw. den zylindrischen Abschnitt 10b verbindet.

Zur Funktionsweise der Vorrichtung wird Folgendes ausgeführt:
Die Vorrichtung gemäß den Fig. 1 bis 6 basiert auf dem Gedanken, das Verschlussmittel 13 insbesondere das selbstverschließende Verschlussmittel 13 mit einer Betätigungseinrichtung 12 zu kombinieren, die es erlaubt, im Gebrauch eine in Richtung des Aufnahmeelements 10 wirkende Kraft auf das zu entfernende Konkrement auszuüben. Damit wird erreicht, dass die Vorrichtung stationär im Gefäß angeordnet ist und das Konkrement auf die Vorrichtung zubewegt und von dieser aufgenommen wird. Die Erfindung ist besonders geeignet für eine Ausführungsform, bei der die Aufbringung der Löse- bzw. Bewegungskraft, die auf das Konkrement wirkt, hydraulisch, d.h. durch einen Unterdruck bzw. durch ein Druckgefälle über den Thrombus hinweg erzeugt wird. Dazu bildet, wie vorstehend beschrieben, das Aufnahmeelement 10 und dessen fluiddichte Abdeckung 14 einen durch das zu entfernende Konkrement verschließbaren, insbesondere proximalen Hohlraum 15.

Das Verschlussmittel 13 ist dem Hohlraum 15 in Strömungsrichtung nachgeordnet bzw. distal zum Hohlraum 15 angeordnet. Damit ist das Verschlussmittel im Gebrauch zwischen dem Hohlraum 15 und dem zu entfernenden Konkrement positionierbar. Der Hohlraum 15 ist insofern durch das Konkrement verschließbar, da das Konkrement, wie in Fig. 2 dargestellt, im Zusammenwirken mit dem Verschlussmittel 13 den Hohlraum 15 des Aufnahmeelements 10 fluiddicht abdichtet. Damit wird erreicht, dass der Hohlraum 15 mit Drücken beaufschlagbar ist, die sich vom physiologischen Druck unterscheiden, insbesondere durch einen Unterdruck bzw. durch einen Überdruck, so dass das gewünschte Druckgefälle am Thrombus erzeugbar ist.

Durch den erzeugten Unterdruck im Korb bzw. im Aufnahmeelement 10 wird der Thrombus in proximaler Richtung durch den distal vom Thrombus herrschenden höheren physiologischen Druck gedrückt. Wenn der Thrombus in Kontakt mit der Vorrichtung, insbesondere mit dem Verschlussmittel 13 gelangt, übt der Thrombus eine nach außen gerichtete radiale Kraft auf das selbstschließende Verschlussmittel 13, insbesondere auf die Verjüngung aus. Die vom Thrombus ausgeübte Kraft kann dabei auch rein axial sein, d.h. ohne radiale Komponente, wobei durch eine entsprechend angepasste Struktur des Korbes bzw. Verschlussmittels 13 eine Ausweitung der Verjüngung 18 hervorgerufen wird. Insbesondere kann der Korb bzw. das Verschlussmittel 13 drahtgeflochten sein, so dass die Ausweitung durch eine reine Axialkomprimierung hervorgerufen wird.

Dabei ist der Thrombus in Fig. 2 im Einlassbereich 13b des Verschlussmittels 13 angeordnet. Wie in Fig. 2 gezeigt, ist der Einlassbereich 13b so groß dimensioniert, dass der Thrombus ohne Widerstand bis zur Verjüngung 18 gleiten kann und dort die radiale Betätigungskraft ausübt. Aufgrund der Druckausgleichsöffnungen 21 herrscht im Außenbereich um die Verjüngung herum der gleiche Druck P2, wie im Hohlraum 15. Da die aufgrund des höheren physiologischen Drucks außerhalb des Hohlraums 15 auf das Konkrement wirkende Kraft entsprechend groß ist, dehnt sich der Korb bzw. das Aufnahmeelement 10 an der verjüngten Stelle bzw. im Bereich der Verjüngung 18 radial nach außen aus. Die Druckausleichsöffnungen 21 können klein sein, da kein signifikanter Volumenstrom durch diese hindurchströmen muss. Dasselbe gilt im Prinzip auch für den Durchmesser der Verjüngung im Ruhezustand, die so ausgelegt ist, das eine Druckübertragung vom proximalen Hohlraum 15 auf den Bereich zwischen Verschlussmittel 13 und Konkrement möglich ist.

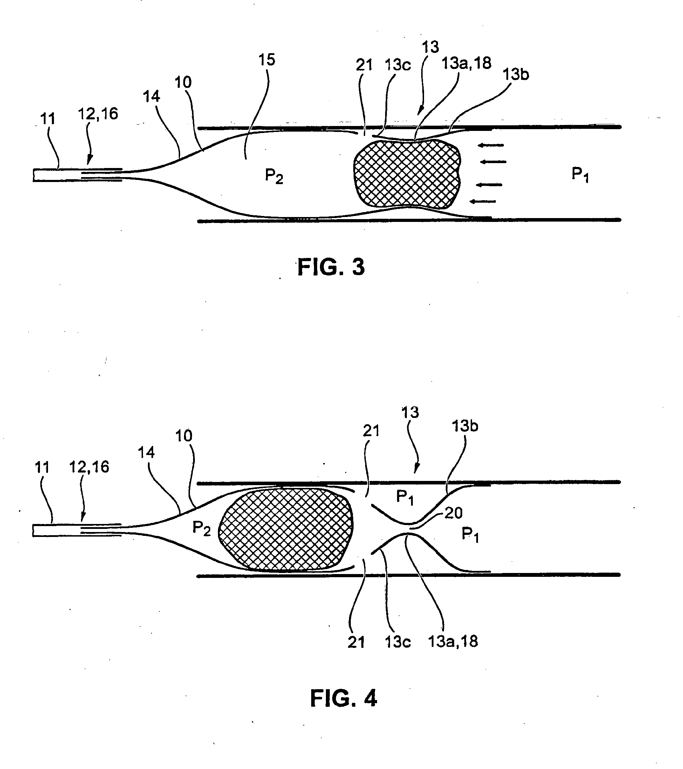

Wie in Fig. 3 dargestellt, kann sich der Thrombus etwas verformen, wenn dieser den aufgeweiteten verjüngten Bereich bzw. die Verjüngung 18 passiert, wie in Fig. 3 dargestellt. Der Thrombus wird dabei in den Korb bzw. in das Aufnahmeelement 10 hineingesaugt. Wenn sich der Thrombus im Korb bzw. im Aufnahmeelement 10 (Fig. 4) befindet, und zwar proximal zu den seitlichen Druckausgleichsöffnungen 21, kommt es zu einer Fluidverbindung zwischen dem Zwischenraum um die Verjüngung 18 herum und dem distalen Bereich hinter dem Verschlussmittel 13. Dadurch stellt sich ein Gleichgewicht der externen Kräfte im Bereich der Verjüngung 18 ein, so dass die Rückstellkraft der Verjüngung 18 bzw. des selbstschließenden Verschlussmittels 13 dazu führt, dass die Vorrichtung ihren Ruhezustand einnimmt.

Die Vorrichtung hat des Weiteren folgende Vorteile: Im Hinblick auf die Partikelablösung ist die Verjüngung 18 des Verschlussmittels 13 so klein dimensioniert, dass keine größeren Blutpartikel aus dem Aufnahmeelement 10 abfließen können. Die Öffnung ist lediglich so zu dimensionieren, dass der Unterdruck im Aufnahmeelement 10 auf den Bereich zwischen dem Aufnahmeelement 10 und dem Thrombus übertragen werden kann, um das Druckgefälle über den Thrombus zu erzeugen. Aufgrund der flexiblen Ausgestaltung der Verjüngung 18 kann die in der Schließstellung kleine Öffnung aufgeweitet werden, um in der Offenstellung einen ausreichend großen Durchgang für den Thrombus zu schaffen. Insofern wird auf die eingangs genannten Werte im Hinblick auf den Innendurchmesser der Verjüngung, das Verhältnis zwischen Innendurchmesser im verjüngten Bereich und im Einlass- bzw. Auslassbereich sowie auf die Querschnittsfläche der Druckausgleichsöffnungen 21 bzw. der Verschlussöffnung 20 verwiesen. Letztere können einander im Wesentlichen entsprechen.

Als Material für den Korb bzw. das Aufnahmeelement wird ein superelastisches Material (NiTi) verwendet, das geschnitten oder, in bevorzugter Variante geflochten ist. Geflochtene Stents werden verkürzt, wenn sie radial ausgedehnt werden, bzw. werden radial ausgedehnt, wenn sie in Längsrichtung, d.h. axial komprimiert werden, was die positiven Eigenschaften der Erfindung unterstützt. Geflochtene Stents können mit vielen, sehr feinen Drähten hergestellt werden, was den Vorteil hat, dass eine sehr feinmaschige Struktur erzielt wird. Die Abdeckung 14 bzw. die Beschichtung kann mit Kunststoff oder durch eine Metallfolie erfolgen. Die Beschichtung kann sich bis zur Verjüngung erstrecken, damit der Unterdruckbereich fluiddicht abgedichtet werden kann. Distal zur Abdeckung 14 kann die Struktur offen bleiben und, aufgrund ihrer Feinmaschigkeit, als Filter die Ablösung bzw. den Austrag von großen Partikeln verhindern. Wenn die Vorrichtung in geflochtener Bauweise hergestellt ist, wird vorteilhafterweise im Bereich der Verjüngung 18 ein kleinerer Flechtwinkel gewählt, wodurch die Verjüngung 18 in radialer Richtung besonders flexibel ist, ohne dabei zu einer signifikanten axialen Komprimierung der Vorrichtung zu führen. Die Flechtwinkel im Bereich der Verjüngung können weniger als 30°, weniger als 20°, weniger als 10°, weniger als 5° betragen.

Die kontinuierliche Querschnittsänderung im Einlass- bzw. Auslassbereich 13b, 13c kann Winkel kleiner 80°, kleiner 70°, kleiner 60°, kleiner als 45°, kleiner als 30° umfassen. Die kontinuierliche Querschnittsänderung ist besonders wirksam am distalen Ende, d.h. am Einlassbereich 13b, um ein möglichst widerstandsfreies Einführen des Thrombus in das Verschlussmittel zu gestatten. Ferner ist die axiale Länge der Verjüngung 18 relativ kurz, insbesondere kürzer als die axiale Länge des Aufnahmeelementes 10 bzw. kürzer als die axiale Länge des Einlassbereichs 13b bzw. des Auslassbereichs 13c. Die axiale Länge des gesamten Verschlussmittels 13, d.h. des Einlassbereichs 13b, der Verjüngung 18 und des Auslassbereichs 13c, kann kleiner als 10 mm, kleiner als 8 mm, kleiner als 5 mm, kleiner als 4 mm, kleiner als 3 mm, kleiner als 2 mm sein. Die für die Ausdehnung der Verjüngung 18 erforderliche Radialkraft wird durch die Flexibilität der Verjüngung 18 bestimmt, wobei vermieden wird, dass der Thrombus nicht zu stark verformt wird. Damit wird erreicht, dass das Risiko der Partikelablösung durch Reibung im Bereich des Verschlussmittels 13 verringert wird. Ein weiterer Vorteil des geflochtenen Stents besteht in der geschlossenen Zellenstruktur, wodurch der Stent bzw. die Vorrichtung besser in einen schlauchartigen Katheter zum Entfernen der Vorrichtung einschließlich des gefangenen Thrombus gezogen werden kann.

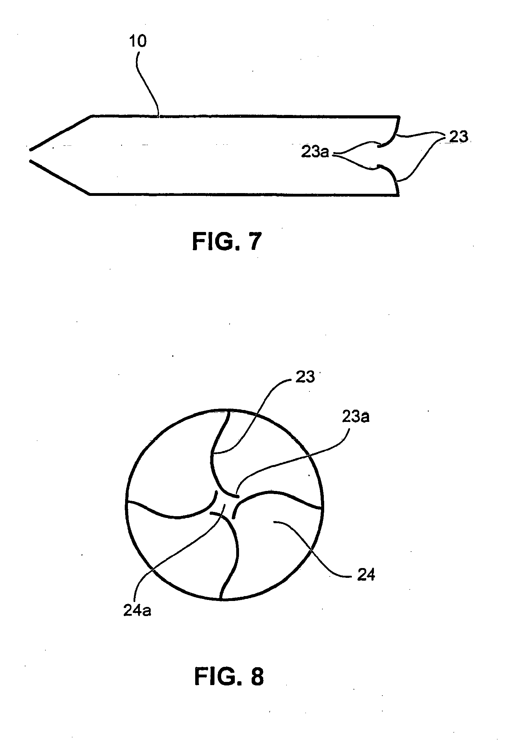

Bei dem Ausführungsbeispiel gemäß den Fig. 7 und 8 wird eine Variante des Verschlussmittels 13 mit einer Betätigungseinrichtung 12 kombiniert, die im Gebrauch eine in Richtung des Aufnahmeelementes 10 wirkende Kraft auf das zu entfernenden Konkrement ausübt. Die Betätigungseinrichtung kann einen mechanisch wirkenden distalen Schirm und/oder eine hydraulisch wirkende Saug- und/oder Druckeinrichtung umfassen. Im Unterschied zu dem Ausführungsbeispiel gemäß den Fig. 1 bis 6 ist das Verschlussmittel 13 gemäß Fig. 7, 8 am distalen Ende des Aufnahmeelementes 10, insbesondere im Bereich der Endkante vorgesehen. Dabei umfasst das Verschlussmittel eine Vielzahl von Drähten 23, die über den Außendurchmesser des Aufnahmeelementes 10 radial nach innen vorstehen. Dabei bilden die Drähte 23 jeweils freie Enden 23a, die in der Ruheposition radial nach innen vorstehen (Fig. 7). Die freien Enden 23a können zusätzlich in proximaler Richtung vorstehen, wobei die freien Enden 23a nach innen in Längsrichtung des Aufnahmeelementes 10 gebogen bzw. gekrümmt sind. Zusätzlich oder alternativ können die freien Enden 23a bzw. die freien Drähte 23 in Umfangsrichtung des Aufnahmeelementes 10 gekrümmt sein (Fig. 8). Dabei werden auch paarweise oder mehrere miteinander verdrillte oder anders verbundene Drähte als Drähte 23 mit freien Enden 23a angesehen.

Die Drähte 23 wirken als Klappe oder Ventil und können mit einer Beschichtung, insbesondere einer Kunststoffbeschichtung, beispielsweise einer Polyurethanbeschichtung versehen sein. Die Beschichtung bildet eine weitere fluiddichte Abdeckung 24, die eine verschließbare Durchtrittsöffnung 24a für das zu entfernende Konkrement aufweist und eine Bewegung der freien Enden 23a der Drähte 23 radial nach außen erlaubt. Dazu ist die Abdeckung 24 in der Ruheposition, d.h. in der Schließstellung des Verschlussmittels 13 so gefaltet, dass die Drähte 23 der Bewegung des zu entfernenden Konkrements in das Aufnahmeelement 10 hinein nachgeben können.

Es ist auch möglich, eine vorgeformte, crimpbare Membran vorzusehen, die in das distale Ende des Korbes bzw. des Aufnahmeelementes 10 eingefügt ist bzw. mit dem distalen Ende des Aufnahmeelementes 10 verbunden ist. Die Membran kann beispielsweise aus Polyurethan bestehen. Die Membran kann mit dem distalen Ende des Korbes durch Kleben oder Heften, beispielsweise durch ein Lösemittel, wenn der Korb zuvor bereits mit Polyurethan beschichtet wurde, verbunden sein.

Allgemein wird ein Aufnahmeelement 10 offenbart, das eine crimpbare Membran aufweist, die mit dem distalen Ende des Aufnahmeelements 10 verbunden ist und eine proximal nach innen gewölbte Form mit einer zentralen Einlassöffnung aufweist. Die Form der Membran kann dem in Fig. 7 dargestellten Querschnitt entsprechen. Die im Zusammenhang mit den nach innen geklappten Drahtenden offenbarten Merkmale werden, soweit sinnvoll, auch im Zusammenhang mit der nach innen geklappten Membran offenbart.

Der kleinere Durchmesser der Membran, d.h. der Durchmesser der Einlassöffnung, kann axial geschlitzt sein, insbesondere mehrfach axial geschlitzt sein, wodurch erreicht wird, das Thromben einfach, d.h. mit einem geringen Widerstand, durch die Membran gezogen werden können und von dieser sicher zurückerhalten werden.

Es ist auch möglich, nach innen umgeformte Schlaufen am distalen Korbende vorzusehen, die einen Winkel von ca. 90° aufweisen. Der Thrombus wird durch die nach innen geneigten Schlaufenenden durch den Unterdruck gezogen und zurückgehalten. Die Schlaufenenden können beschichtet sein. Die am distalen Ende des Korbes bzw. des Aufnahmeelements 10 vorgesehenen umgeschlagenen Korbschlaufen können unterschiedlichen Längen aufweisen. Ferner ist es denkbar, beispielsweise die Schlaufen teilweise durch eine geeignete Wärmebehandlung nach innen zu klappen. Beispielsweise könnte jede zweite Schlaufe per Wärmebehandlung nach innen geklappt sein. Beim Crimpen werden die Schlaufen gestreckt, wodurch vermieden wird, dass sich die doppelte Wandstärke für die Korbstruktur bzw. die Struktur des Aufnahmeelements 10 aufbaut.

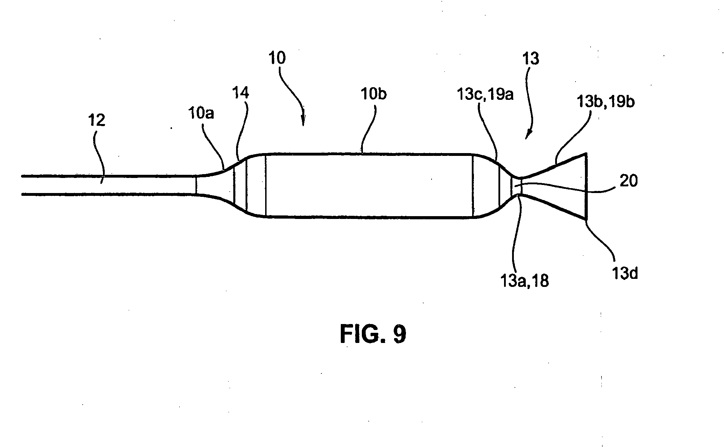

In Fig. 9 ist ein weiteres Ausführungsbeispiel der erfindungsgemäßen Vorrichtung dargestellt, wobei das Verschlussmittel 13 doppeltrichterförmig ausgebildet ist. Die Vorrichtung gemäß dem Ausführungsbeispiel nach Fig. 9 weist also im Wesentlichen dieselbe Struktur auf, wie bereits im Zusammenhang mit Fig. 1 beschrieben. Der Unterschied besteht darin, dass die trichterartigen Abschnitte 19a, 19b unterschiedliche Trichterwinkel umfassen. Insbesondere ist durch den proximalen trichterartigen Abschnitt 19a ein proximaler Auslassbereich 13c gebildet, der mit der Rotationsachse der Vorrichtung einen größeren Winkel einschließt als der durch den distalen trichterartigen Abschnitt 19b gebildete distale Einlassbereich 13b. Der Einlassbereich 13b verjüngt sich somit in Richtung der Verjüngung 18 bzw. des Verschlussbereichs 13a, insbesondere der Verschlussöffnung 20, unter einem flacheren Winkel als der distale Auslassbereich 13c. Der distale Endabschnitt 13d, d.h. die Grundfläche des trichterartigen Abschnitts 19b, der den Einlassbereich 13b bildet, entspricht dem Querschnittsdurchmesser des zylindrischen Abschnitts 10b des Aufnahmeelements 10. Dieser Querschnittsdurchmesser entspricht auch dem Durchmesser der Grundfläche des proximalen trichterartigen Abschnitts 13c. Die unterschiedlichen Trichterwinkel zwischen den beiden trichterartigen Abschnitten 19a, 19b sind dadurch gebildet, dass die beiden trichterartigen Abschnitte 19a, 19b eine unterschiedliche axiale Längserstreckung aufweisen. Insbesondere ist der proximale trichterartige Abschnitt 19a in axialer Richtung kürzer als der distale trichterartige Abschnitt 19b. Es ist möglich, dass das Verhältnis der Trichterwinkel der beiden trichterartigen Abschnitte 19a, 19b umgekehrt ist. In diesem Fall weist der proximale trichterartige Abschnitt 19a einen kleineren Trichterwinkel auf als der distale trichterartige Abschnitt 19b. Die Variante mit einem distalen trichterartigen Abschnitt 19b bzw. einen distalen Einlassbereich 13b, der einen kleineren Trichterwinkel aufweist, ist bevorzugt, da auf diese Weise die Entfernung eines Konkrements, insbesondere eines Thrombus, erleichtert ist. Dieser Effekt wird dadurch erreicht, dass durch den flacheren Trichterwinkel des distalen Einlassbereichs 13b durch den in proximaler Richtung bewegten, insbesondere gesogenen bzw. aspirierten, Thrombus eine kleinere Kraftkomponente auf die Verjüngung 18 aufgebracht werden muss, um die Verschlussöffnung 20 soweit aufzuweiten, dass der Thrombus hindurchtreten kann.

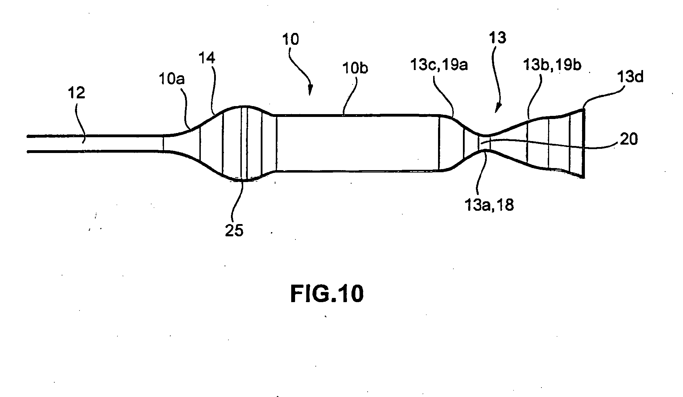

Wie in Fig. 10 dargestellt, ist es ferner möglich, dass der distale trichterartige Abschnitt 19b, insbesondere der distale Einlassbereich 13b trompetenartig geformt ist. Zur Bildung der Trompetenform ist der distale Endabschnitt 13d in distaler Richtung aufgeweitet bzw. radial nach außen geformt. Der distale Endabschnitt 13d weist einen größeren Querschnittsdurchmesser auf als der zylindrische Abschnitt 10b des Aufnahmeelements 10. Auf diese Weise wird der distale Endabschnitt 13d im Gebrauch stärker gegen die Gefäßwand gespannt, so dass zwischen der Gefäßwand und dem distalen Einlassbereich 13b im Wesentlichen ein kontinuierlicher bzw. fließender Übergang bereitgestellt wird. Dadurch wird verhindert, dass das Konkrement bzw. der Thrombus beim Bewegen in den Einlassbereich 13b durch die Drahtenden im distalen Endabschnitt 13d beschädigt wird oder sich im distalen Endabschnitt 13d verhakt. Ferner kann der distale Endabschnitt 13d einen Schneidebereich bilden, so dass durch eine Rotationsbewegung und/oder eine Axialbewegung in distale Richtung ein an der Gefäßwand festsitzendes Konkrement, beispielsweise ein Thrombus oder ein Plaque, von der Gefäßwand gelöst bzw. getrennt werden kann. Der Schneidebereich ist vorzugsweise derart ausgebildet, dass eine Verletzung der Gefäßwand vermieden wird. Beispielsweise können die distal offenen Drahtenden des distalen Endabschnitts 13d schlaufenartig geformt sind, so dass scharfe Kanten bzw. Drahtenden im Bereich des Endabschnitts 13d vermieden werden. In den Schlaufen bzw. allgemein im Endabschnitt 13d können Röntgenmarker angeordnet sein. Röntgenmarker umfassen Materialien, die eine relativ hohe Dichte bzw. Röntgensichtbarkeit aufweisen. Die Röntgenmarker können in Form von Hülsen in die Drahtschlaufen des distalen Endabschnitts 13d eingeflochten sein.

Bei dem Ausführungsbeispiel gemäß Fig. 10 ist im proximalen Bereich des Aufnahmeelements 10 ferner ein proximaler Dichtungsabschnitt 25 vorgesehen, der einen größeren Querschnittsdurchmesser aufweist als der zylindrische Abschnitt 10b des Aufnahmeelements 10. Insbesondere kann der proximale Dichtungsabschnitt 25 im Wesentlichen denselben Querschnittsdurchmesser aufweisen wie der distale Endabschnitt 13d des trompetenartig geformten distalen trichterartigen Abschnitts 19b des Verschlussmittels 13. Es wird darauf hingewiesen, dass die in Fig. 10 dargestellte Kombination des proximalen Dichtungsabschnitts 25 und dem trompetenförmig ausgebildeten distalen trichterartigen Abschnitt 13b eine bevorzugte Variante der Vorrichtung darstellt. Es ist jedoch nicht ausgeschlossen, dass die Vorrichtung einen proximalen Dichtungsabschnitt 25 ohne ein trompetenartig geformtes Verschlussmittel 13 aufweist. Umgekehrt ist es ebenfalls möglich, dass bei der Vorrichtung ein trompetenartiges Verschlussmittel 13 vorgesehen ist, ohne dass das Aufnahmeelement 10 einen proximalen Dichtungsabschnitt 25 aufweist.

Der proximale Dichtungsabschnitt 25 ist im Wesentlichen durch eine Auswölbung des Aufnahmeelements 10 im Übergangsbereich zwischen dem zylindrischen Abschnitt 10b und dem trichterförmigen Abschnitt 10a gebildet. Der proximale Dichtungsabschnitt 25 weist eine relativ flexible Struktur auf, so dass sich der Querschnittsdurchmesser des proximalen Dichtungsabschnitts 25 beim Schieben des Betätigungselements 12 in distale Richtung erhöht. Der proximale Dichtungsabschnitt 25 weitet sich also bei Bewegung des Betätigungselements 12 in distale Richtung radial auf. Dabei dichtet der proximale Dichtungsabschnitt 25 im Gebrauch die Vorrichtung gegen die Gefäßwand ab, wobei gleichzeitig die Kontaktfläche zwischen Aufnahmeelement 10 und Gefäßwand reduziert wird. Beim Entfernen der Vorrichtung aus dem Körpergefäß, d.h. beim Ziehen der Vorrichtung, insbesondere des Betätigungselements 12, in proximale Richtung, wird der proximale Dichtungsabschnitt 25 axial gedehnt, wodurch eine Verringerung des Querschnittsdurchmessers des proximalen Dichtungsabschnitts 25 erreicht wird. Dadurch wird gewährleistet, dass die Scherkräfte, die beim Gleiten des Aufnahmeelements 10 entlang der Gefäßwand auf die Gefäßwand wirken, reduziert werden. Die Vorrichtung weist vorzugsweise eine fluiddichte Abdeckung 14 auf, die sich zumindest über den trichterförmigen Abschnitt 10a erstreckt. Besonders bevorzugt ist es, wenn sich die fluiddichte Abdeckung 14 über das gesamte Aufnahmeelement 10 bzw. die gesamte Vorrichtung, d.h. das Aufnahmeelement 10 und das Verschlusselement 13, erstreckt.

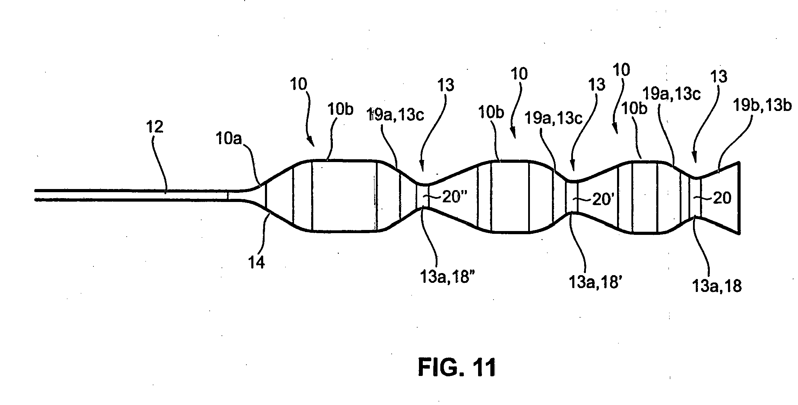

Gemäß einem weiteren Ausführungsbeispiel kann die Vorrichtung, wie in Fig. 11 dargestellt, mehrere, insbesondere drei Verjüngungen 18, 18', 18" aufweisen, die jeweils einen Verschlussbereich 13a bilden. Die Verschlussbereiche 13a weisen jeweils eine Verschlussöffnung 20, 20', 20" auf, wobei eine erste Verschlussöffnung 20 gegenüber den weiteren Verschlussöffnungen 20', 20" einen größeren Querschnittsdurchmesser aufweist. Der Querschnittsdurchmesser der Verschlussöffnungen 20, 20', 20" wird mit zunehmendem Abstand zum distalen Einlassbereich 13b kleiner. Im Wesentlichen weist die Vorrichtung gemäß dem Ausführungsbeispiel nach Fig. 11 also drei oder mehrere Aufnahmeelemente 10 auf, denen jeweils ein Verschlussmittel 13 zugeordnet ist, wobei die Verschlussmittel 13 unterschiedlich große Verjüngungen 18, 18', 18" aufweisen. Die kaskadenartige Anordnung von Aufnahmeelementen 10 bzw. Verschlussmitteln 13 ermöglicht ein besonders schonendes Entfernen von Konkrementen. Insbesondere wird bei der Aufnahme eines Konkrements bzw. eines Thrombus der Thrombus je nach seiner Größe in eines der Aufnahmeelemente 10 aufgenommen. Kleinere Thromben erreichen dabei das am weitesten proximal angeordnete Aufnahmeelement 10, wohingegen größere Thromben in einem mittleren oder distalen Aufnahmeelement 10 fixiert werden. Die Aufnahmeelemente 10 und die Verschlussmittel 13 umfassen eine fluiddichte Abdeckung, die sich vollständig über die Aufnahmeelemente 10 und die Verschlussmittel 13 erstreckt. Die flexible Abdeckung 14 begrenzt den Hohlraum, in dem der Druck, insbesondere Unterdruck, zur Entfernung des Thrombus bzw. Konkrements wirkt. Wenn die Vorrichtung im Bereich von Gefäßverzweigungen eingesetzt bzw. positioniert ist, wird mit der flexiblen Abdeckung 14 verhindert, dass die abzweigenden, d.h. kollateralen, Gefäße mit dem erzeugten Druck beaufschlagt werden. Da die Vorrichtung gemäß Fig. 11 mehrere Verjüngungen 18, 18', 18" aufweist, ist die Kontaktfläche zwischen der Vorrichtung und dem Blutgefäß reduziert, so dass eine Beschädigung oder Beeinträchtigung der Gefäßwand verringert ist.

Die in den Figuren 10 und 11 dargestellten Ausführungsbeispiele weisen jeweils ein oder mehrere Verschlussmittel 13 auf, die doppeltrichterförmig ausgebildet sind. Dabei ist der Trichterwinkel des jeweils distalen trichterartigen Abschnitts 19b kleiner als der Trichterwinkel des jeweils proximalen trichterartigen Abschnitts 19a. Es ist auch möglich, dass die beiden trichterartigen Abschnitte 19a, 19b jeweils gleiche Trichterwinkel aufweisen. Bevorzugte Materialien für die Herstellung der erfindungsgemäßen Vorrichtung sind beispielsweise Edelstahl, Formgedächtnismaterialien oder Kunststoffe, insbesondere Kunststofffasern. Die Vorrichtung kann aus einem rohrförmigen Rohmaterial bzw. Halbzeug beispielsweise durch Laserschneiden oder andere geeignete Verfahren hergestellt sein. Es ist ebenfalls möglich, dass die Vorrichtung eine geflochtene Struktur aufweist, d.h. mehrere Drahtelemente umfasst, die miteinander verflochten, verdrillt oder verwoben sind. Die bevorzugten Materialen und Herstellungsformen können miteinander kombiniert sein.

### Bezugszeichenliste

- 10: Aufnahmeelement
- 10a: trichterförmiger Abschnitt
- 10b: zylindrischer Abschnitt
- 11: Zufuhreinrichtung
- 12: Betätigungselement
- 13: Verschlussmittel
- 13a: Verschlussbereich
- 13b: Einlassbereich
- 13c: Auslassbereich
- 13d: Endabschnitt
- 14: fluiddichte Abdeckung
- 15: Hohlraum
- 16: Saug- und/oder Druckeinrichtung
- 18, 18', 18": Verjüngung
- 19a, 19b: trichterartige Abschnitte
- 19c: Halsbereich
- 20, 20', 20": Verschlussöffnung
- 21: Druckausgleichsöffnung
- 22: Stützelement
- 23: Drähte
- 23a: freie Enden
- 24: weitere fluiddichte Abdeckung
- 25: proximaler Dichtungsabschnitt

## Patentansprüche

1. Vorrichtung zum Entfernen von Konkrementen aus Körpergefäßen mit wenigstens einem komprimierbaren Aufnahmeelement (10) zur Aufnahme von Konkrementen und einer Zufuhreinrichtung (11) zur Führung des komprimierten Aufnahmeelements (10), wobei dem Aufnahmeelement (10) eine Betätigungseinrichtung (12) und ein Verschlussmittel (13) zugeordnet sind, und das Verschlussmittel (13) in eine Offenstellung zum Einführen des Konkrements in das Aufnahmeelement (10) und in eine Schließstellung zum Rückhalten des Konkrements im Aufnahmeelement (10) überführbar ist, *wobei* die Betätigungseinrichtung (12) angepasst ist derart, dass diese im Gebrauch eine in Richtung des Aufnahmeelementes (10) wirkende Kraft auf das zu entfernende Konkrement ausübt, wobei
*das Aufnahmeelement (10) eine fluiddichte Abdeckung (14) aufweist, die einen durch das zu entfernende Konkrement verschließbaren, insbesondere proximalen Hohlraum (15) bildet, wobei die Betätigungseinrichtung (12) im Gebrauch mit der Abdeckung (14) und*/*oder dem Hohlraum (15) in Wirkverbindung steht derart, dass im Hohlraum (15) zum Bewegen, insbesondere zum Lösen des Konkrements ein anderer Druck einstellbar ist als außerhalb des Hohlraums (15), **dadurch gekennzeichnet, dass** das Verschlussmittel (13) einen selbstschließenden Verschlussbereich (13a) aufweist, der zwischen einem distalen Einlassbereich (13b) und einem proximalen Auslassbereich (13c) des Verschlussmittels (13) angeordnet ist, wobei der Einlassbereich (13b) zur Aufnahme des zu entfernenden Konkrements angepasst und der Auslassbereich (13c) zum Aufnahmeelement (10) hin geöffnet ist.*

2. Vorrichtung nach Anspruch *1,*
**dadurch gekennzeichnet, dass**
das Betätigungselement (12) eine Saug- und/oder Druckeinrichtung (16) umfasst, die mit dem Hohlraum (15) fluidverbunden oder fluidverbindbar ist

3. Vorrichtung nach Anspruch *1* oder *2*,
**dadurch gekennzeichnet, dass**
das Betätigungselement (12) einen distal zum Konkrement positionierbaren Schirm (17) umfasst, der in Richtung des Aufnahmeelements (10) bewegbar ist.

4. Vorrichtung nach *wenigstens einem der Ansprüche 1 bis 3,*
**dadurch gekennzeichnet, dass**
das Verschlussmittel (13), insbesondere der Verschlussbereich (13a) zumindest abschnittsweise eine Verjüngung (18) mit einem veränderbaren Querschnittsdurchmesser umfasst, wobei die Verjüngung im Ruhezustand einen kleineren Querschnittsdurchmesser aufweist als der Einlassbereich (13b) des Verschlussmittels (13).

5. Vorrichtung nach Anspruch 4,
**dadurch gekennzeichnet, dass**
das Verschlussmittel (13) doppeltrichterförmig ausgebildet ist, wobei zwei trichterartige Abschnitte (19a, 19b) miteinander verbunden sind und im Halsbereich (19c) zwischen den Abschnitten die Verjüngung (18) bilden.

6. Vorrichtung nach Anspruch 4 oder 5,
**dadurch gekennzeichnet, dass**
das Verschlussmittel (13) im Bereich der Verjüngung (18) eine Verschlussöffnung (20) bildet, die mit dem Aufnahmeelement (10) fluchtet, insbesondere zum Aufnahmeelement (10) koaxial angeordnet ist.

7. Vorrichtung nach wenigstens einem der Ansprüche 4 bis 6,
**dadurch gekennzeichnet, dass**
das Verhältnis des Innendurchmessers der Verjüngung (18) zum Innendurchmesser des Einlassbereichs (13b) im Ruhezustand höchstens 0,8, insbesondere höchstens 0,5, insbesondere höchstens 0,1, insbesondere höchstens 0,05, insbesondere höchstens 0,02 beträgt.

8. Vorrichtung nach wenigstens einem der Ansprüche 4 bis 7,
**dadurch gekennzeichnet, dass**
das Verschlussmittel (13) zumindest im Bereich der Verjüngung (18) wenigstens eine Druckausgleichsöffnung (21) aufweist.

9. Vorrichtung nach wenigstens einem der Ansprüche *1* bis 8,
**dadurch gekennzeichnet, dass**
sich die fluiddichte Abdeckung (14) des Aufnahmeelementes (10) mindestens bis vor den Auslassbereich (13c) erstreckt.

10. Vorrichtung nach wenigstens einem der Ansprüche 4 bis 9,
**dadurch gekennzeichnet, dass**
das Verschlussmittel (13) im Wesentlichen eine geflochtene, insbesondere stent-ähnliche, Gitterstruktur umfasst, *wobei die Gitterstruktur im Bereich der Verjüngung (18) im Ruhezustand einen kleineren Flechtwinkel aufweist als im Bereich des Aufnahmeabschnitts (10).*

11. Vorrichtung nach wenigstens einem der Ansprüche *1* bis *10*,
**dadurch gekennzeichnet, dass**
das Verschlussmittel (13) im Einlassbereich (13a) und/oder im Auslassbereich (13c) eine kontinuierliche Querschnittsänderung aufweist.

12. Vorrichtung nach wenigstens einem der Ansprüche 4 bis 11,
**dadurch gekennzeichnet,dass**
dem Verschlussmittel (13) ein Stützelement (22) zugeordnet ist, das radial außen bezogen auf das Verschlussmittel (13) angeordnet ist und im Gebrauch eine Gefäßwand des Körpergefäßes zumindest im Bereich der Verjüngung (18) stützt.

13. Vorrichtung nach wenigstens einem der Ansprüche 1 bis 12,
**dadurch gekennzeichnet, dass**
das Verschlussmittel (13) eine Vielzahl von Drähten (23) mit freien Enden (23a) umfasst, die am distalen Ende des Aufnahmeelements (10) angeordnet sind und in der Ruheposition radial nach Innen vorstehen.

14. Vorrichtung nach Anspruch 13,
**dadurch gekennzeichnet, dass**
die freien Enden (23a) in proximaler Richtung vorstehen und/oder in Umfangsrichtung des Aufnahmeelementes (10) gekrümmt sind.

15. Vorrichtung nach Anspruch 13 oder 14,
**dadurch gekennzeichnet, dass**
die freien Enden (23a) mit einer weiteren fluiddichten Abdeckung (24), insbesondere einer flächigen Beschichtung verbunden sind, die eine Durchtrittsöffnung (24a), insbesondere eine verschließbare Durchtrittsöffnung (24a) für das zu entfernende Konkrement bildet und eine Bewegung der freien Enden (23a) radial nach Außen erlaubt.

## Claims

1. Device for removing concretions from vessels of the body, having at least one compressible receiving element (10) for receiving concretions and a feed device (11) for guiding the compressed receiving element (10), the receiving element (10) being associated with an actuating device (12) and a closure means (13), and the closure means (13) being movable into an open position for introducing the concretion into the receiving element (10) and into a closed position for retaining the concretion in the receiving element (10), wherein the actuating device (12) is adapted in such a way that, in use, it exerts on the concretion to be removed a force acting in the direction of the receiving element (10), wherein the receiving element (10) has a fluid-tight covering (14) which forms a cavity, especially a proximal cavity (15), closable by the concretion to be removed, wherein in use the actuating device (12) is in operative connection with the covering (14) and/or with the cavity (15) in such a way that for moving, especially for detaching, the concretion the pressure in the cavity (15) can be set to a pressure different from that outside the cavity (15), **characterised in that** the closure means (13) has a self-closing closure region (13a) which is arranged between a distal inlet region (13b) and a proximal outlet region (13c) of the closure means (13), wherein the inlet region (13b) is adapted to receive the concretion to be removed and the outlet region (13c) is open towards the receiving element (10).

2. Device according to claim 1,
**characterised in that**
the actuating element (12) comprises a suction and/or pressure device (16) which is in fluidic connection or can be brought into fluidic connection with the cavity (15).

3. Device according to claim 1 or 2,
**characterised in that**
the actuating element (12) comprises an umbrella (17) positionable distally with respect to the concretion, which umbrella is movable in the direction of the receiving element (10).

4. Device according to at least one of claims 1 to 3,
**characterised in that**
the closure means (13), especially the closure region (13a), comprises, at least in some regions, a tapered portion (18) of variable cross-sectional diameter, wherein the tapered portion has in the rest position a smaller cross-sectional diameter than the inlet region (13b) of the closure means (13).

5. Device according to claim 4,
**characterised in that**
the closure means (13) is of double-funnel-like shape, wherein two funnel-like sections (19a, 19b) are connected to one another and form the tapered portion (18) in the neck region (19c) between the sections.

6. Device according to claim 4 or 5,
**characterised in that**
the closure means (13) forms in the region of the tapered portion (18) a closure opening (20) which is in alignment with the receiving element (10), especially arranged coaxially with the receiving element (10).

7. Device according to at least one of claims 4 to 6,
**characterised in that**
the ratio of the internal diameter of the tapered portion (18) to the internal diameter of the inlet region (13b) in the rest position is at most 0.8, especially at most 0.5, especially at most 0.1, especially at most 0.05, especially at most 0.02.

8. Device according to at least one of claims 4 to 7,
**characterised in that**
the closure means (13), at least in the region of the tapered portion (18), has at least one pressure-equalisation opening (21).

9. Device according to at least one of claims 1 to 8,
**characterised in that**
the fluid-tight covering (14) of the receiving element (10) extends at least up to in front of the outlet region (13c).

10. Device according to at least one of claims 4 to 9,
**characterised in that**
the closure means (13) primarily comprises a braided, especially stent-like, lattice structure, wherein in the rest position the lattice structure has a smaller braiding angle in the region of the tapered portion (18) than in the region of the receiving portion (10).

11. Device according to at least one of claims 1 to 10,
**characterised in that**
the closure means (13) exhibits a continuous change in cross-section in the inlet region (13a) and/or in the outlet region (13c).

12. Device according to at least one of claims 4 to 11,
**characterised in that**
the closure means (13) is associated with a support element (22) which is arranged radially outside relative to the closure means (13) and in use supports a vessel wall of the vessel of the body at least in the region of the tapered portion (18).

13. Device according to at least one of claims 1 to 12,
**characterised in that**
the closure means (13) comprises a multiplicity of wires (23) having free ends (23a) which are arranged at the distal end of the receiving element (10) and project radially inwards in the rest position.

14. Device according to claim 13,
**characterised in that**
the free ends (23a) project in the proximal direction and/or are curved in the circumferential direction of the receiving element (10).

15. Device according to claim 13 or 14,
**characterised in that**
the free ends (23a) are connected to a further fluid-tight covering (24), especially a planar coating, which forms a passageway (24a), especially a closable passageway (24a), for the concretion to be removed and allows movement of the free ends (23a) radially outwards.

## Revendications

1. Dispositif servant à supprimer des concrétions des vaisseaux du corps, comprenant au moins un élément de réception (10) compressible servant à recevoir des concrétions et comprenant un système d'amenée (11) servant à guider l'élément de réception (10) comprimé, sachant qu'un système d'actionnement (12) et un moyen d'obturation (13) sont associés à l'élément de réception (10) et que le moyen d'obturation (13) peut passer dans une position d'ouverture afin d'introduire la concrétion dans l'élément de réception (10) et dans une position de fermeture afin de maintenir la concrétion dans l'élément de réception (10), sachant que le système d'actionnement (12) est adapté de telle manière qu'il exerce, lorsqu'il est utilisé, une force sur la concrétion à supprimer, laquelle force agissant en direction de l'élément de réception (10), sachant que
l'élément de réception (10) présente un élément de recouvrement (14) étanche aux fluides, lequel forme une cavité (15) pouvant être obturée par la concrétion à supprimer, en particulier une cavité proximale, sachant que le système d'actionnement (12) est en relation fonctionnelle, lorsqu'il est utilisé, avec l'élément de recouvrement (14) et/ou avec la cavité (15), qu'une pression autre que la pression régnant à l'extérieur de la cavité (15) peut être ajustée dans la cavité (15) afin de déplacer, en particulier de défaire la concrétion, **caractérisé en ce que** le moyen d'obturation (13) présente une zone d'obturation (13a) à fermeture automatique, laquelle est disposée entre une zone d'admission distale (13b) et une zone d'évacuation proximale (13c) du moyen d'obturation (13), sachant que la zone d'admission (13b) est adaptée aux fins de la réception de la concrétion à supprimer et que la zone d'évacuation (13c) est ouverte en direction de l'élément de réception (10).

2. Dispositif selon la revendication 1,
**caractérisé en ce que**
l'élément d'actionnement (12) comprend un système d'aspiration et/ou de pression (16), qui est relié ou peut être relié de manière fluidique à la cavité (15).

3. Dispositif selon la revendication 1 ou 2,
**caractérisé en ce que**
l'élément d'actionnement (12) comprend un écran de protection (17) pouvant être positionné de manière distale par rapport à la concrétion, lequel écran peut être déplacé en direction de l'élément de réception (10).

4. Dispositif selon au moins l'une quelconque des revendications 1 à 3,
**caractérisé en ce que**
le moyen d'obturation (13), en particulier la zone d'obturation (13a) comprend au moins par endroits un rétrécissement (18), le diamètre de la section transversale duquel variant, sachant que la section transversale du rétrécissement présente, au repos, un diamètre de plus petite taille que celui de la zone d'admission (13b) du moyen d'obturation (13).

5. Dispositif selon la revendication 4,
**caractérisé en ce que**
le moyen d'obturation (13) est réalisé de manière à présenter une forme de double cône, sachant que deux sections (19a, 19b) se présentant à la manière de cônes sont reliées l'une à l'autre et forment dans la zone de col (19c) entre les sections le rétrécissement (18).

6. Dispositif selon la revendication 4 ou 5,
**caractérisé en ce que**
le moyen d'obturation (13) forme dans la zone du rétrécissement (18) un orifice d'obturation (20) qui est aligné avec l'élément de réception (10), qui est disposé en particulier de manière coaxiale par rapport à l'élément de réception (10).

7. Dispositif selon au moins l'une quelconque des revendications 4 à 6,
**caractérisé en ce que**
le rapport entre le diamètre intérieur du rétrécissement (18) et le diamètre intérieur de la zone d'admission (13b) au repos est au maximum de 0,8, en particulier au maximum de 0,5, en particulier au maximum de 0,1, en particulier au maximum de 0,05, en particulier au maximum de 0,02.

8. Dispositif selon au moins l'une quelconque des revendications 4 à 7,
**caractérisé en ce que**
le moyen d'obturation (13) présente au moins dans la zone du rétrécissement (18) au moins un orifice de compensation de pression (21).

9. Dispositif selon au moins l'une quelconque des revendications 1 à 8,
**caractérisé en ce que**
l'élément de recouvrement (14) étanche aux fluides de l'élément de réception (10) s'étend au moins jusque devant la zone d'évacuation (13c).

10. Dispositif selon au moins l'une quelconque des revendications 4 à 9,
**caractérisé en ce que**
le moyen d'obturation (13) comprend essentiellement une structure tressée, en particulier une structure similaire à celle d'un stent, une structure maillée, sachant que la structure maillée présente, dans la zone du rétrécissement (18) au repos un angle de tressage de taille plus petite que celui de la zone de la section de réception (10).

11. Dispositif selon au moins l'une quelconque des revendications 1 à 10,
**caractérisé en ce que**
le moyen d'obturation (13) présente, dans la zone d'admission (13a) et/ou dans la zone d'évacuation (13c), un changement continu de la section transversale.

12. Dispositif selon au moins l'une quelconque des revendications 4 à 11,
**caractérisé en ce**
**qu'**un élément de soutien (22) est associé au moyen d'obturation (13), lequel élément de soutien est disposé radialement à l'extérieur par rapport au moyen d'obturation (13) et soutient, lorsqu'il est utilisé, une paroi de vaisseau du vaisseau du corps au moins dans la zone du rétrécissement (18).

13. Dispositif selon au moins l'une quelconque des revendications 1 à 12,
**caractérisé en ce que**
le moyen d'obturation (13) comprend une pluralité de fils (23) pourvus d'extrémités libres (23a), lesquels sont disposés au niveau de l'extrémité distale de l'élément de réception (10) et font saillie radialement vers l'intérieur en position de repos.

14. Dispositif selon la revendication 13,
**caractérisé en ce que**
les extrémités libres (23a) font saillie dans la direction proximale et/ou sont courbées dans la direction périphérique de l'élément de réception (10).

15. Dispositif selon la revendication 13 ou 14,
**caractérisé en ce que**
les extrémités libres (23a) sont reliées à un autre élément de recouvrement (24) étanche aux fluides, en particulier à un revêtement plan, qui forme un orifice de passage (24a), en particulier un orifice de passage (24a) pouvant être obturé pour la concrétion à supprimer et autorise un déplacement des extrémités libres (23a) radialement vers l'extérieur.
